# EUROPEAN PATENT APPLICATION

(11) **EP 4 206 176 A1**
(43) Date of publication of application: **05.07.2023**
(21) Application number: 21861962.5
(22) Date of filing: 17.08.2021
(51) Int. Cl.: C07C 45/61, C07C 45/46, C07C 49/784, B01J 27/10, C08K 5/132, C08L 71/12

(54) **METHOD FOR PREPARING 1,4-BIS(4-PHENOXYBENZOYL)BENZENE AND 1,4-BIS(4-PHENOXYBENZOYL)BENZENE PREPARED THEREBY**

(30) Priority: 28.08.2020 KR 20200108990
(71) Applicant: Hanwha Solutions Corporation, Jung-gu Seoul 04541 (KR)
(72) Inventor: PARK, Seong Min, Daejeon 34128 (KR); SEO, Min Guk, Daejeon 34128 (KR); JUNG, Ki Taeg, Daejeon 34128 (KR)
(74) Representative: Klunker IP Patentanwälte PartG mbB
(86) International application number: PCT/KR2021/010911
(87) International publication number: WO 2022/045665

(57) **Abstract**

The present invention relates to a method for preparing 1,4-bis(4-phenoxybenzoyl)benzene. According to the present invention, when a 1,4-bis(4-phenoxybenzoyl)benzene synthesis reaction is carried out at a specific temperature, the amount of heat used in the preparing process can be minimized while maintaining the same yield as that of the existing preparing method. In addition, waste solvent generated in the preparing process does not undergo a color change and thus can be reused, and thus energy saving effects can be provided.

## Description

### TECHNICAL FIELD

The present invention relates to a method for preparing 1,4-bis(4-phenoxybenzoyl)benzene. According to the present invention, when a 1,4-bis(4-phenoxybenzoyl)benzene synthesis reaction is carried out at a specific temperature, the amount of heat used in the preparing process can be minimized while maintaining the same yield as that of the existing preparing method. In addition, waste solvent generated in the preparing process does not undergo a color change and thus can be reused, thereby providing an energy saving effect.

### BACKGROUND ART

Polyether ketone is a generic term for already known industrial resins, and includes polyether ketone, polyether ether ketone (PEEK), polyether ketone ketone (PEKK), and a copolymer in which polyether ketone ketone (PEKK) and polyether ketone are partially mixed.

Since polyether ketone ketone (PEKK) has high heat resistance and excellent strength, PEKK is widely used as engineering plastics. Engineering plastics are used in the fields of automobiles, aircraft, electrical and electronic equipment, machines, and the like, and their field of applications is gradually expanding.

Diphenyl ether, terephthaloyl halide, isophthaloyl halide, and the like are used as monomers for the polymerization of PEKK, and 1,4-bis(4-phenoxybenzoyl)benzene (EKKE) is used as an intermediate in the polymerization process.

In particular, 1,4-bis(4-phenoxybenzoyl)benzene may be polymerized through a Friedel-Crafts Acylation chain reaction that is an electrophilic substitution reaction between an aromatic acid chloride and an aromatic ether in the presence of Lewis acids.

Referring to related technologies, US Patent No. 4,918,237 relates to a polymerization process of 1,4-bis(4-phenoxybenzoyl)benzene and discloses a method of employing 1,4-bis(4-phenoxybenzoyl)benzene using a specific metal-containing catalyst.

Korean Patent Laid-Open No. 10-2020-0007871 discloses a method for preparing 1,4-bis(4-phenoxybenzoyl)benzene by using terephthaloyl chloride, diphenyl ether, solvent, and Lewis acid, and in particular, discloses a technique for providing purity grades so as to obtain a solution in which terephthaloyl chloride has a specific turbidity at a specific temperature and a specific concentration.

Korean Patent Laid-Open No. 10-2020-0009038 discloses a method for preparing 1,4-bis(4-phenoxybenzoyl)benzene by using terephthaloyl chloride, diphenyl ether, solvent, and Lewis acid, and in particular, discloses a technology for recovering 1,4-bis(4-phenoxybenzoyl)benzene by centrifugal filtration in the process of separating 1,4-bis(4-phenoxybenzoyl)benzene prepared after a synthesis reaction.

As described above, the technology for the method for preparing 1,4-bis(4-phenoxybenzoyl)benzene is being developed in various ways. The present invention has been completed as part of research to improve productivity and manufacturing efficiency of 1,4-bis(4-phenoxybenzoyl)benzene as a reactant or intermediate for preparing polyether ketone being in high demand as an industrial resin today.

### DESCRIPTION OF EMBODIMENTS

### TECHNICAL PROBLEM

The present invention aims to solve the above-described problems.

The present invention aims to perform a synthesis reaction at a specific temperature in a process of preparing 1,4-bis(4-phenoxybenzoyl)benzene to minimize the amount of heat used in the preparing process while maintaining the same yield as that of the existing preparing method, thereby providing an energy saving effect.

In addition, the present invention aims to enable reuse of a solvent used in a reaction process because the solvent does not undergo a color change after a neutralization reaction.

### SOLUTION TO PROBLEM

In order to achieve the above-described objects of the present invention and realize the characteristic effects of the present invention described below, the characteristic configuration of the present invention is as follows.

An embodiment of the present invention provides a method for preparing 1,4-bis(4-phenoxybenzoyl)benzene, the method including the steps of: (a) preparing a solution by dissolving terephthaloyl chloride (TPC) and diphenyl ether (DPO) in a solvent; (b) cooling the solution to -10°C to -5°C and maintaining the temperature of the solution; (c) preparing a mixed solution by adding a catalyst to the solution, and performing a synthesis reaction by raising the temperature of the mixed solution to 20°C to 40°C; (d) desorbing the catalyst while cooling and maintaining the temperature to 1°C to 15°C after the synthesis; and (e) obtaining 1,4-bis(4-phenoxybenzoyl)benzene by separating 1,4-bis(4-phenoxybenzoyl)benzene and waste solvent from the mixed solution.

In particular, the waste solvent in the step (e) has an APHA value of 1 to 10 and a yellow index (YI) value of 0.1 to 1 after neutralization and filtering.

Another embodiment of the present invention provides 1,4-bis(4-phenoxybenzoyl)benzene prepared by the preparing method.

### ADVANTAGEOUS EFFECTS OF DISCLOSURE

According to the present invention, a synthesis reaction is performed at a specific temperature in a process of preparing 1,4-bis(4-phenoxybenzoyl)benzene to minimize the amount of heat used in the preparing process while maintaining the same yield as that of the existing preparing method, thereby providing an energy saving effect.

In addition, according to the present invention, since a solvent used in a reaction process does not undergo a color change after a neutralization reaction, the solvent can be reused, thereby helping save energy.

### BRIEF DESCRIPTION OF DRAWING

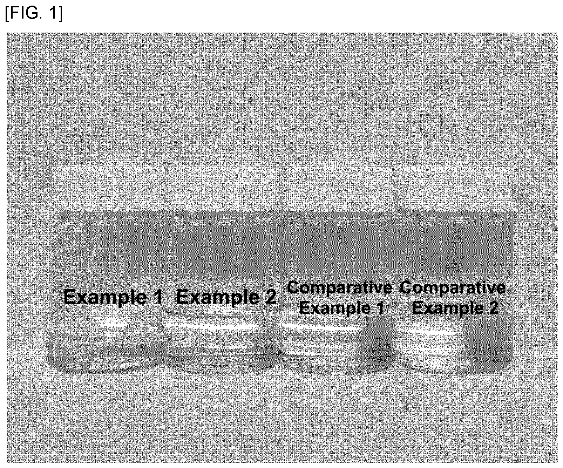

FIG. 1 is a visual observation of colors of solvents after neutralization of waste solvent generated in preparing processes of Examples and Comparative Examples.

### BEST MODE

Hereinafter, the structure and operation of the present invention will be described in more detail with reference to preferred examples of the present invention. However, these examples are shown by way of illustration and should not be construed as limiting the present invention in any sense.

Since contents not described herein can be sufficiently technically inferred by those of ordinary skill in the art, descriptions thereof will be omitted.

### Example 1

A solution was prepared by dissolving 30.16 g of terephthaloyl chloride (TPC) and 126.28 g of diphenyl ether (DPO) in 676 g of ortho-dichlorobenzene and was cooled to -5°C, and a mixed solution was prepared by adding 81.7 g of aluminum chloride while maintaining the temperature below -5°C. The temperature of the mixed solution was raised to 20°C, and Friedel-Crafts Acylation was performed as a synthesis reaction. After the synthesis reaction, the mixed solution was cooled to 10°C, and a catalyst was desorbed by adding 500 ml of methanol for 60 minutes while maintaining the temperature. After the desorption, the mixed solution on which the synthesis reaction was completed was filtered and separated from waste solvent to obtain 1,4-bis(4-phenoxybenzoyl)benzene. On the other hand, NaOH was added to the waste solvent so as to remove AlCl₃ contained therein, and salts and fine particles were removed through filtering.

### Example 2

Example 2 was performed under the same conditions as in Example 2, except that the temperature of the mixed solution was raised to 30°C.

### Comparative Example 1

Comparative Example 1 was performed under the same conditions as in Example 1, except that the temperature of the mixed solution was raised to 40°C.

### Comparative Example 2

Comparative Example 2 was performed under the same conditions as in Example 1, except that the temperature of the mixed solution was raised to 60°C.

### Experimental Example 1

The amounts of heating and cooling were measured in order to compare the energy consumption and the yield of 1,4-bis(4-phenoxybenzoyl)benzene (EKKE) according to the synthesis temperatures in Examples and Comparative Examples. In addition, APHA and YI were measured in order to measure the degree of color change of waste solvent for reuse. The results thereof are shown in Table 1. In addition, a result of visually comparing the colors after neutralization of the waste solvent is shown in FIG. 1.

APHA and YI measurements were performed by using a Vista spectrophotometer (available from HunterLab) and followed color standard analysis methods (ASTM E1164 and ASTM E 313).

**[Table 1]**

| | Synthesis temperature °C | EKKE wt% | Amount of heating kJ | Amount of cooling kJ | APHA-10mm | YI E313 |
|---|---|---|---|---|---|---|
| Example 1 | 20 | 99.8 | 27.3 | 10.9 | 7.77 | 0.53 |
| Example 2 | 30 | 99.8 | 38.2 | 21.9 | 8.67 | 0.56 |
| Comparative Example 1 | 40 | 99.8 | 49.2 | 32.8 | 65.62 | 3.75 |
| Comparative Example 1 | 60 | 99.8 | 71.0 | 54.6 | 129.94 | 7.35 |

As described in Table 1 above, in the case of Examples in which the temperature for synthesizing the reactants, that is, terephthaloyl chloride (TPC), diphenyl ether, and the catalyst according to the present invention was limited to 20°C to 30°C, the amount of heating and cooling was significantly reduced while constantly maintaining the yield of 1,4-bis(4-phenoxybenzoyl)benzene (EKKE),compared to Comparative Examples. Accordingly, an energy saving effect can be expected in the preparing process.

In addition, when the waste solvent generated in the preparing process is separated and neutralized, it can be confirmed that the APHA value is significantly low compared to Comparative Examples, and it can be confirmed that it is close to a transparent resin in light of the numerical values.

In addition, it can be confirmed that the YI value is also significantly low compared to Comparative Examples, and it can be confirmed that the values of Examples are relatively good for contamination or chemical exposure in light of the numerical values. It can be confirmed that this means that the waste solvent can be reused.

While the present invention has been described by particular matters such as specific components and limited embodiments and drawings, this is provided only for helping the comprehensive understanding of the present invention. The present invention is not limited to the above-described embodiments, and it will be understood by those of ordinary skill in the art that various modifications and variations can be made thereto without departing from the scope of the present invention.

Therefore, it will be understood that the spirit of the present invention should not be limited to the above-described embodiments and the claims and all equivalent modifications fall within the scope of the present invention.

### MODE OF DISCLOSURE

Reference is made to the accompanying drawing which shows, by way of illustration, specific embodiments in which the present invention may be practiced. The embodiments will be described in detail in such a manner that the present invention can be carried out by those of ordinary skill in the art. It should be understood that various embodiments of the present invention are different from each other, but need not be mutually exclusive. For example, certain shapes, structures, and features described herein may be implemented in other embodiments without departing from the spirit and scope of the present invention in connection with one embodiment. In addition, it will be understood that the locations or arrangement of individual components in the disclosed embodiments can be changed without departing from the spirit and scope of the present invention. Therefore, the following detailed description is not to be taken in a limiting sense, and the scope of the present invention is to be limited only by the appended claims and the entire scope of equivalents thereof, if properly explained. Like reference numerals in the drawing refer to the same or similar functions throughout the various aspects.

Hereinafter, preferred embodiments of the present invention will be described in detail with reference to the accompanying drawing, so that those of ordinary skill in the art can easily carry out the present invention.

The present invention intends to provide a method for preparing 1,4-bis(4-phenoxybenzoyl)benzene represented by Formula 1 below and 1,4-bis(4-phenoxybenzoyl)benzene prepared thereby.

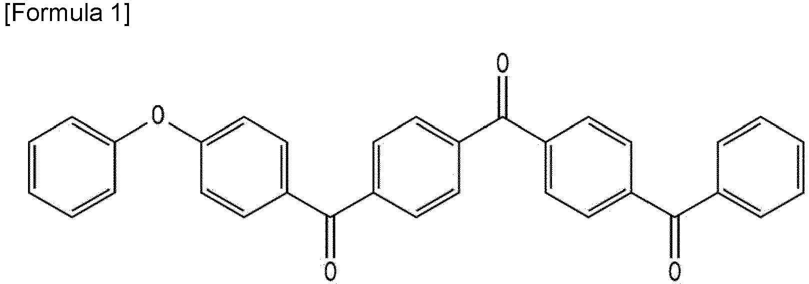

The present invention provides a method for preparing 1,4-bis(4-phenoxybenzoyl)benzene, including the steps of: (a) preparing a solution by dissolving terephthaloyl chloride (TPC) and diphenyl ether (DPO) in a solvent; (b) cooling the solution to -10°C to -5°C and maintaining the temperature of the solution; (c) preparing a mixed solution by adding a catalyst to the solution, and performing a synthesis reaction by raising the temperature of the mixed solution to 20°C to 40°C; (d) desorbing the catalyst while cooling and maintaining the temperature to 1°C to 15°C after the synthesis; and (e) obtaining 1,4-bis(4-phenoxybenzoyl)benzene by separating 1,4-bis(4-phenoxybenzoyl)benzene and waste solvent from the mixed solution.

In particular, in the present invention, the synthesis reaction of 1,4-bis(4-phenoxybenzoyl)benzene is performed in the preparing process, and the amount of heating used in the preparing process is minimized while maintaining the same yield as the existing preparing method. The solvent used in the reaction process does not undergo a color change after neutralization and filtering, thereby enabling the solvent to be reused.

First, the step (a) of preparing a solution by dissolving terephthaloyl chloride (TPC) represented by Formula 2 below and diphenyl ether (DPO) represented by Formula 3 below in a solvent is provided.

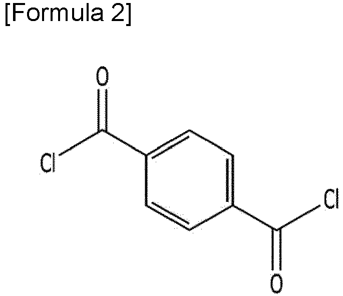

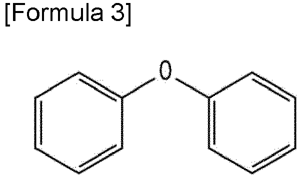

In the step (a), the weight ratio of terephthaloyl chloride (TPC) to diphenyl ether (DPO) may be 1:1 to 10. Preferably, a weight ratio of terephthaloyl chloride (TPC) to diphenyl ether (DPO) may be 1:1 to 3. A high yield of 1,4-bis(4-phenoxybenzoyl)benzene can be obtained within the above range. When a very large amount of diphenyl ether is used, a lot of load occurs in a cleaning and separation process.

In addition, the solvent provided in the step (a) may be at least one selected from ortho-dichlorobenzene and dichloromethane. Any solvent may be used as long as the solvent is commonly used as a solvent for Friedel-Crafts Acylation polymerization reaction, but the present invention is not limited thereto.

Next, the step (b) of cooling the solution to -10°C to -5°C and maintaining the temperature of the solution is provided. The catalyst is added after lowering the temperature of the solution. At this time, the temperature of the solution may vary depending on the catalyst, but is preferably -10°C to -5°C.

Next, the step (c) of preparing a mixed solution by adding a catalyst to the solution, and performing a synthesis reaction by raising the temperature of the mixed solution to 20°C to 40°C is provided. Preferably, the temperature of the mixed solution may be 20°C to 30°C.

In this case, the synthesis reaction refers to Friedel-Crafts Acylation. In addition, the provided catalyst acts as a Lewis acid, and at least one selected from aluminum chloride (AlCl₃), potassium carbonate (K₂CO₃), and iron chloride (FeCl₃) may be provided. In addition, any catalyst may be used as long as the catalyst is commonly used as a Lewis acid for Friedel-Crafts Acylation, but the present invention is not limited thereto.

In particular, in the present invention, the temperature of the synthesis reaction may be 20°C to 40°C, and preferably 20°C to 30°C. Within the above range, the amount of heating used in the preparing process is minimized while maintaining the same yield as the existing preparing method, and the solvent used in reaction process does not undergo a color change after neutralization, thereby enabling reuse. The results thereof can be referred to in Table 1 and FIG. 1 to be described below.

In addition, the synthesis reaction may be performed for 2 hours to 10 hours. The synthesis reaction may be carried out in various reactors, For example, the synthesis reaction may be carried out in a batch reactor, a continuous stirred tank reactor (CSTR), or a loop reactor. The pressure may be 1 bar (atmospheric pressure) to 5 bar, and preferably 1 bar (atmospheric pressure).

Next, the step (d) of desorbing the catalyst while cooling and maintaining the temperature of the mixed solution to 1°C to 15°C is provided.

The desorption preferably means removing the catalyst. More specifically, the catalyst may be removed by adding at least one selected from methanol, acetic acid, formic acid, ethanol, isopropanol, and benzyl alcohol to the mixed solution for 30 minutes to 120 minutes. Preferably, the removal of the catalyst may be performed by adding methanol (CHsOH) for 60 minutes to 90 minutes.

Next, the step (e) of obtaining 1,4-bis(4-phenoxybenzoyl)benzene by separating 1,4-bis(4-phenoxybenzoyl)benzene and the waste solvent from the mixed solution is provided. 1,4-bis(4-phenoxybenzoyl)benzene precipitates as a solid in the mixed solution and exists in the solution. This solution may be filtered to separate and obtain 1,4-bis(4-phenoxybenzoyl)benzene, and the waste solvent may be separately recovered.

In the case of the filtration, batch filtration or continuous filtration may be provided. For example, the filtration may be performed by a pressure filter, a Nutsche filter, a candle filter, a centrifugal separator, and the like, but the present invention is not limited thereto.

In addition, 1,4-bis(4-phenoxybenzoyl)benzene is separated by the filtration, and the recovered waste solvent is neutralized by adding a basic solution such as sodium hydroxide (NaOH). Thereafter, the remaining solution is filtered to remove aluminum hydroxide (Al(OH)₃), sodium chloride (NaCl), and fine particles. In the case of the filtering, a Nutsche filter, a candle filter, a centrifugal separator, and the like, which are commonly used, may be used. After the filtering, a step of washing with deionized water (DI water) may be further included if necessary.

Accordingly, the waste solvent may have an APHA value of 10 or less after neutralization and filtering, and is characterized in that the APHA value is 1 to 10.

APHA color is also referred to as Hazen scale or cobalt (Pt/Co) scale and is a color standard analysis method (ASTM E1164) named for the American Public Health Association. A color of a sample to be measured is analyzed by APHA value. Therefore, the APHA value of the waste solvent generated in the preparing process according to the present invention may provide the color of the solvent close to transparent.

In addition, the separated waste solvent has a YI (yellow index) value of 0.1 to 1 after neutralization and filtering.

The YI value is a numerical expression of the degree of darkness and lightness of yellow, and may be measured by using a colorimeter. It is related to burning, contamination, or general deterioration caused by process, light, chemical exposure, and the yellowness of the sample to be measured may be analyzed by an analysis method (ASTM E 313). Therefore, the waste solvent generated in the preparing process according to the present invention has a YI value of 1 or less, thereby providing relatively good physical property values with respect to contamination or chemical exposure.

Therefore, in light of the APHA value and the YI value described above, the waste solvent can be separated using a distillation column and thus reused. The distillation column may be performed by, for example, batch distillation or continuous distillation. The batch distillation is a method in which raw materials are put into a distillation kiln and distilled without additional addition, and reflux is first performed and partial reflux is then performed in a normal state to extract an effluent.

The continuous distillation uses a tray column or a packing column to continuously supply raw materials at an appropriate location in the middle of the column. Components with low boiling points flow out from the top of the column, and components with high boiling points flow out from the bottom of the column. In this manner, each component is extracted continuously.

Accordingly, as described above, the waste solvent that has undergone neutralization and filtering may be separated and reused by extracting unnecessary components through batch distillation or continuous distillation.

On the other hand, the present invention provides 1,4-bis(4-phenoxybenzoyl)benzene prepared according to the preparing method described above. Accordingly, it can be provided as a reaction intermediate of polyether ketone, which is in high demand as an industrial resin. The amount of heating used in the preparing process is minimized while maintaining the same yield as the existing preparing method, and the waste solvent generated in the preparing process does not undergo a color change and thus can be reused, thereby providing an energy saving effect.

In addition, the present invention provides a polymer resin including 1,4-bis(4-phenoxybenzoyl)benzene.

In this case, the polymer resin may include at least one selected from polyether ketone, polyether ether ketone, and polyether ketone ketone. The polymer resin including the materials described above may be used as engineering plastics. A plastic material prepared by including the polymer resin may be provided as a vehicle material, an electronic device material, an industrial material, a construction engineering material, a 3D printer material, a textile material, a cladding material, a machine tool material, a medical material, an aviation material, a photovoltaic material, a battery material, a sports material, a household appliance material, a household material, and a cosmetic material.

In a more specific example, a product including the plastic material may be an automotive air duct, a plastic and rubber compound, an adhesives, a light, a polymer optical fiber, a fuel filter cap, a line system, a cable for an electronic device, a reflector, a cable sheath, an optical fiber, a wire protection tube, a control unit, a light, a tube for pipe, a liner, a pipe coating agent, an oil field exploration hose, a 3D printer, a multi-filament, a spray hose, a valve, a duct, a pulp, a gear, a medical catheter, a flame retardant for aircraft, a protective plate for a solar cell, cosmetics, a high-hardness film, ski boots, a headset, a glasses frame, a toothbrush, a water bottle, or outsole, but the present invention is not limited thereto.

### INDUSTRIAL APPLICABILITY

According to the present invention, a synthesis reaction is carried out at a specific temperature in a process of preparing 1,4-bis(4-phenoxybenzoyl)benzene to minimize the amount of heat used in the preparing process while maintaining the same yield as that of the existing preparing method, thereby providing an energy saving effect.

In addition, according to the present invention, since a solvent used in a reaction process does not undergo a color change after a neutralization reaction, the solvent can be reused, thereby helping save energy.

## Claims

1. A method for preparing 1,4-bis(4-phenoxybenzoyl)benzene, the method comprising the steps of:
(a) preparing a solution by dissolving terephthaloyl chloride (TPC) and diphenyl ether (DPO) in a solvent;
(b) cooling the solution to -10°C to -5°C and maintaining the temperature of the solution;
(c) preparing a mixed solution by adding a catalyst to the solution, and performing a synthesis reaction by raising the temperature of the mixed solution to 20°C to 40°C;
(d) desorbing the catalyst while cooling and maintaining the temperature to 1°C to 15°C after the synthesis; and
(e) obtaining 1,4-bis(4-phenoxybenzoyl)benzene by separating 1,4-bis(4-phenoxybenzoyl)benzene and waste solvent from the mixed solution.

2. The method of claim 1, wherein a weight ratio of the terephthaloyl chloride (TPC) to the diphenyl ether (DPO) in the step (a) is 1:1 to 10.

3. The method of claim 1, wherein the solvent in the step (a) is at least one selected from ortho-dichlorobenzene and dichloromethane.

4. The method of claim 1, wherein the catalyst in the step (c) is at least one selected from aluminum chloride (AlCl₃), potassium carbonate (K₂CO₃), and iron chloride (FeCl₃).

5. The method of claim 1, wherein the synthesis reaction in the step (c) is Friedel-Crafts Acylation.

6. The method of claim 1, wherein the synthesis reaction in the step (c) is performed for 2 hours to 10 hours.

7. The method of claim 1, wherein the step (d) of desorbing the catalyst is performed by adding at least one selected from methanol, acetic acid, formic acid, ethanol, isopropanol, and benzyl alcohol to the mixed solution.

8. The method of claim 7, wherein the adding is performed for 30 minutes to 90 minutes.

9. The method of claim 1, wherein the waste solvent in the step (e) has an APHA value of 1 to 10 after neutralization and filtering.

10. The method of claim 1, wherein the waste solvent in the step (e) has a yellow index (YI) value of 0.1 to 1 after neutralization and filtering.

11. 1,4-bis(4-phenoxybenzoyl)benzene prepared by the method according to any one of claims 1 to 10.

12. A polymer resin comprising the 1,4-bis(4-phenoxybenzoyl)benzene of claim 11.

13. The polymer resin of claim 12, wherein the polymer resin comprises at least one selected from polyether ketone, polyether ether ketone, and polyether ketone ketone.

14. A plastic material prepared by including the polymer resin of claim 12.

15. The plastic material of claim 14, wherein the plastic material is at least one selected from a vehicle material, an electronic device material, an industrial file material, a construction engineering material, a 3D printer material, a textile material, a cladding material, a machine tool material, a medical material, an aviation material, a photovoltaic material, a battery material, a sports material, a household appliance material, a household material, and a cosmetic material.
